# EUROPÄISCHE PATENTSCHRIFT

(11) **EP 1 834 631 B1**
(45) Veröffentlichungstag und Bekanntmachung des Hinweises auf die Patenterteilung: **09.06.2010**
(21) Anmeldenummer: 07003714.8
(22) Anmeldetag: 23.02.2007
(51) Int. Cl.: A61K 8/63, A61Q 17/04, A61Q 19/00, A61Q 19/04, A61Q 19/08, A61K 8/39, A61K 8/60, A61K 8/37

(54) **Kosmetische oder dermatologische Zubereitungen enthaltend Glycyrrhetin und/oder Glycyrrhizin sowie Glucosederivate bzw. Glycerinether**
Cosmetic or dermatological preparations containing glycyrrhetin and/or glycyrrhizin and glucose derivatives or glycerin ethers
Préparations cosmétiques ou dermatologiques contenant de la glycyrrhétine et/ou de la glycyrrhizine et des dérivés de glucose ou des ethers de glycérine

(30) Priorität: 01.03.2006 DE 102006009852
(43) Veröffentlichungstag der Anmeldung: 19.09.2007
(73) Patentinhaber: Beiersdorf AG, 20253 Hamburg (DE)
(72) Erfinder: Raschke, Thomas, Dr., 25421 Pinneberg (DE); Wolber, Rainer, Dr., 23397 Hamburg (DE); Scherner, Cathrin, Dr., 22455 Hamburg (DE); Cerv, Svenja, 22761 Hamburg (DE); Gerwat, Wolfram, 22309 Hamburg (DE)

(56) Entgegenhaltungen:
- WO-A-01/70188
- WO-A-03/030859
- DE-A1- 10 238 450
- FR-A1- 2 832 061
- US-A- 5 560 917
- ABE, HIROKO ET AL: "Effects of glycyrrhizin and glycyrrhetinic acid on growth and melanogenesis in cultured B16 melanoma cells" EUROPEAN JOURNAL OF CANCER & CLINICAL ONCOLOGY , 23(10), 1549-55 CODEN: EJCODS; ISSN: 0277-5379, 1987, XP002431559
- LEE, JONGSUNG ET AL: "Glycyrrhizin induces melanogenesis by elevating a cAMP level in B16 melanoma cells" JOURNAL OF INVESTIGATIVE DERMATOLOGY , 124(2), 405-411 CODEN: JIDEAE; ISSN: 0022-202X, 2005, XP002431560
- H. EGGENSPERGER: "Multiaktive Wirkstoffe für Kosmetika" 2000, VERLAG FÜR CHEMISCHE INDUSTRIE, H. ZIOLKOWSKY GMBH , AUGSBURG , XP002431821 1. Auflage, Band II, S. 197-214 ISBN 3-87846-213-1 * das ganze Dokument *
- GOTTSCHALCK T.E.; MCEWEN G.N. JR.: 'International Cosmetic Ingredient Dictionary and Handbook', 2004, THE COSMETIC, TOILETRY AND FRAGRANCE ASSOCIATION, WASHINGTON, D.C., ISBN 1-882621-34-4 ISBN 1-882621-34-4 10. Auflage, Band 2, Seite 1427 * Spalte 2 *
- DATABASE REGISTRY 19 August 1994 'TEGO Care 450'

## Beschreibung

Die vorliegende Erfindung betrifft kosmetische und dermatologische Zubereitungen zur Bräunung der Haut, insbesondere solche welche gleichzeitig Schutz gegen UV-Strahlung bieten.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist.
Der schädigende Einfluß der UVB-Strahlung kann durch UVA-Strahlung verstärkt werden.

UV-A-Strahlung kann ferner Hautschädigungen hervorrufen, indem das hauteigene Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeiqt, daß offenbar auch Schädigungen der Erbinformationen in den Zellen durch Sonnenlicht, speziell durch UV-A-Strahlung, hervorgerufen werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Die Pigmentierung der menschlichen Haut, wird im wesentlichen durch die Gegenwart von Melanin bewirkt. Melanin und dessen Abbauprodukte (Melanoide), Carotin, Durchblutungsgrad sowie die Beschaffenheit und Dicke des Stratum corneum und andere Hautschichten lassen Hautfarbtöne von praktisch weiß (bei verringerter Füllung oder bei Fehlen der Blutgefäße) oder gelblich über hellbraun-rötlich, bläulich bis braun verschiedener Nuancen und schließlich beinahe schwarz erscheinen. Die einzelnen Hautregionen zeigen aufgrund unterschiedlicher Melanin-Mengen unterschiedliche Tiefe der Farbtönung.

Das natürliche Melanin schützt die Haut vor eindringender UV-Strahlung. Die Anzahl der in den Melanocyten produzierten Melanin-Granula entscheidet über Hell- od. Dunkelhäutigkeit. Bei starker Pigmentierung (z.B. bei Farbigen, aber auch bei Hellhäutigen nach einiger UV-Bestrahlung) ist Melanin auch im Stratum spinosum und sogar im Stratum corneum festzustellen. Es schwächt die UV-Strahlung um bis zu ca. 90%, bevor diese das Corium erreicht.

Je nach Lichtempfindlichkeit werden in der Regel folgende Hauttypen unterschieden:
Hauttyp I bräunt nie, bekommt immer einen Sonnenbrand.
Hauttyp II bräunt kaum, bekommt leicht einen Sonnenbrand.
Hauttyp III bräunt durchschnittlich gut.
Hauttyp IV bräunt leicht und anhaltend, bekommt fast nie Sonnenbrand.
Hauttyp V dunkle, oft fast schwarze Haut, bekommt nie Sonnenbrand.

Die natürliche Abschirmung der schädlichen UV-Strahlung ist ein handfester Vorteil der natürlichen Hautbräunung. Seit einigen Jahrzehnten gilt darüberhinaus eine "gesunde" Hautfarbe als Zeichen von insbesondere sportlicher Aktivität und wird daher von einer breiten Verbraucherschicht als erstrebenswert erachtet. Vertreter der Hauttypen I und II, die sich einer solchen Hauttönung erfreuen wollen, sind daher ohnehin auf selbstbräunende Präparate angewiesen. Aber auch Vertreter des Hauttyps III, die sich nicht allzusehr den Risiken des Sonnenbades aussetzen und trotzdem gebräunt aussehen wollen, sind dankbare Zielgruppen für selbstbräunende Zubereitungen.

Künstliche Hautbräunung läßt sich auf kosmetischem bzw. medzinischem Wege bewirken, wobei im wesentlichen folgende Ansätze eine Rolle spielen:

Durch regelmäßige Einnahme von Carotin-Präparaten wird Carotin wird im Unterhaut-Fettgewebe gespeichert, die Haut färbt sich allmählich orange bis gelbbraun.

Mit Hilfe abwaschbarer Make-up-Präparate kann eine leichte Hauttönung erzielt werden (z.B. Extrakte aus frischen grünen Walnußschalen, Henna)

Die Anfärbung kann auch auf dem Wege der chemischen Veränderung der Hornschicht der Haut mit sogenannten selbstbräunenden Zubereitungen erfolgen. Wichtigster Wirkstoff ist das Dihydroxyaceton (DHA). Die auf diese Weise erzielte Hautbräunung ist nicht abwaschbar und wird erst mit der normalen Abschuppung der Haut (nach ca. 10-15 Tagen) entfernt. Dihydroxyaceton kann als Ketotriose bezeichnet werden und reagiert als reduzierender Zucker mit den Aminosäuren der Haut bzw. den freien Amino- und Imino-Gruppen des Keratins über eine Reihe von Zwischenstufen im Sinne einer Maillard-Reaktion zu braungefärbten Stoffen, sogenanten Melanoiden, welche gelegentlich auch Melanoidine genannt werden.

Ein Nachteil der Bräunung mit Dihydroxyaceton liegt darin, daß die mit ihm gebräunte Haut im Gegensatze zu "sonnengebräunter" Haut nicht gegen Sonnenbrand geschützt ist.

Glycyrrhetinsäure ist durch folgende Struktur gekennzeichnet:

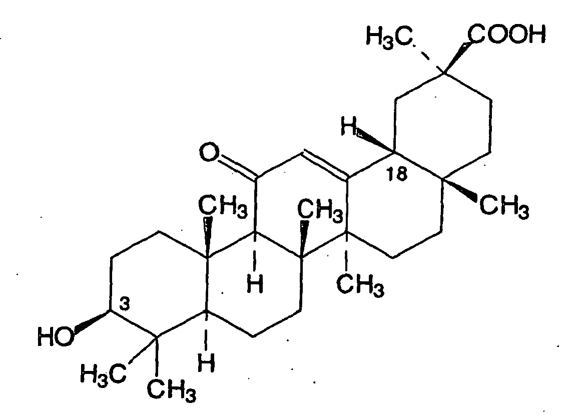

Neben der natürlich vorkommenden 18 β-Form existiert auch eine 18 α-Form. Die aus Süßholz (*Glycyrrhiza glabra*, Leguminosae) isolierbare Glycyrrhetinsäure ist das Aglycon von Glycyrrhizin (welches das 2 β-Glucuronido-a-glucuronid der Glycyrrhetinsäure darstellt).

Glycyrrhizin ist durch folgende Struktur gekennzeichnet:

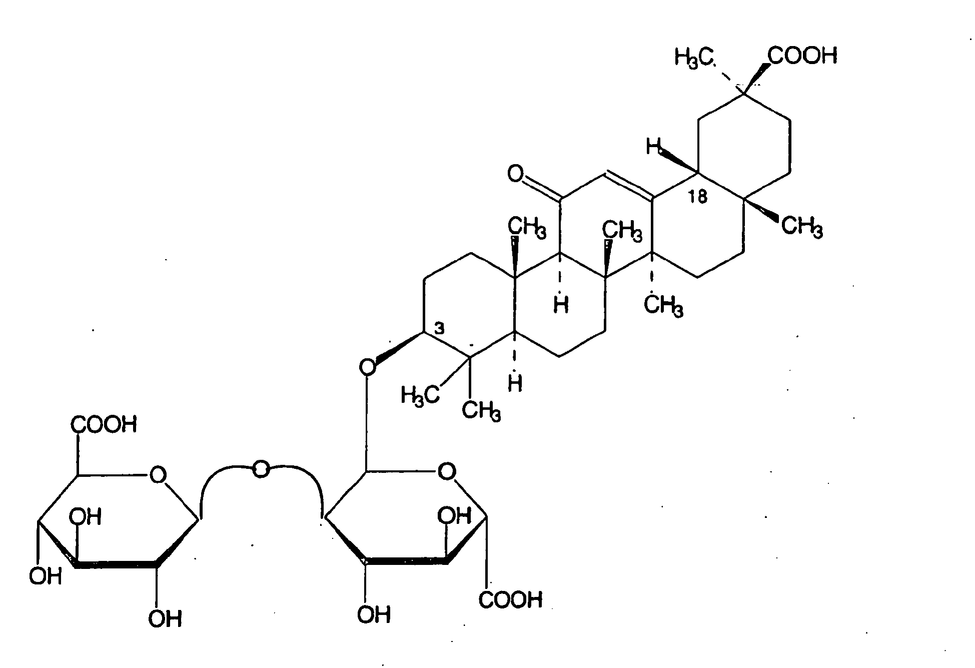

Glycyrrhetinsäure wird als entzündungshemmendes Mittel verwendet. Das Glycyrrhetinsäure-3-Hemisuccinat dient als Ulcustherapeutikum.

Glycyrrhizin wirkt entzündungshemmend und dient in Form von Süßholzsaft (Sirupus liquiritiae) als Hustenmittel und Expektorans.

Nachteilig ist jedoch, daß Glycyrrhizin und Glycyrrhetinsäuren in wasserhaltigen Produkten leicht auskristallisieren, wobei Kristalle mit unkosmetischer Anmutung entstehen und die Wirksamkeit des Produktes vermindert wird. Diese Kristallisationsneigung wird verstärkt durch wasserlösliche Stoffe, deren Solubilisierung Wasser bindet, das somit nicht mehr zur Solubilisierung von Glycyrrhizin und Glycyrrhetinsäuren zur Verfügung steht.

Aufgabe der vorliegenden Erfindung war es somit, Wege zu finden, die die Nachteile des Standes der Technik vermeiden.

Es war überraschend und darin liegt die Lösung dieser Aufgaben, daß die kosmetische Zubereitungen enthaltend
(i) Glycyrrhizin und/oder Glycyrrhetinsäure und
(ii) Polyglycerol (3)-Methylglucosedistearat den Nachteilen des Standes der Technik abhelfen würde.

Vorteilhaft ist insbesondere, wenn die Zubereitungen 0,0001 bis 0,5 Gew.-%, insbesondere 0,001 bis 0,5 Gew.-%, ganz besonders 0,005 bis 0,50 Gew.-% Glycyrrhizin und/oder Glycyrrhetinsäure enthalten, bezogen auf das Gesamtgewicht der Zubereitung. "Polyglyceryl(3)-Methylglucosedistearat" ist unter der Warenbezeichnung Tego Care® 450 von der Gesellschaft Th. Goldschmidt KG erhältlich.

Die Zubereitungen gemäß der Erfindung sind in jeglicher Hinsicht überaus befriedigende Präparate, die sich durch eine hervorragende Wirkung auszeichnen.

Es war für den Fachmann nicht vorauszusehen gewesen, daß die erfindungsgemäßen Zubereitungen
■ die Pigmentierung der Haut steigern.
■ eine gleichmäßigere Bräunung der Haut ermöglichen
■ keine unerwünschten Gerüche beim Auftragen auf die Haut entwickeln
■ eine Verfärbung der Kleidung nach Auftragen auf die Haut vermeiden
■ lichtstrapazierte Haut oder
■ von der Rasur strapazierte Haut besser pflegen,
■ die Nachreaktionen der Haut auf die Einwirkung von UV-Strahlung besser vermindern,
■ die vom Sonnenbaden gereizte Haut besser beruhigen,
■ leichten Sonnenbrand schneller zum Abklingen bringen würden,
■ besser als feuchtigkeitsspendende Zubereitungen wirken,
■ einfacher zu formulieren sein,
■ besser die Hautglättung fördern und als die Zubereitungen des Standes der Technik.

Gegenstand der Erfindung ist daher ferner die Verwendung kosmetischer oder dermatologischer Formulierungen mit einem Gehalt an
(i) Glycyrrhizin und/oder Glycyrrhetinsäure und Polyglyceryl (3) Methylglucosedistearat Zur Herstellung von Zubereifüngen zur Verstärkung der Hautpigmentierung, zur Steigerung der Hautbräunung.

Es ist bevorzugt im Sinne der vorliegenden Erfindung, wenn die erfindungsgemäßen kosmetischen oder dermatologischen Zubereitungen einen oder mehrere Alkohole enthalten, insbesondere, wenn die Formulierungen in Form eines Aftersun-Präparats vorliegen und sich durch eine besondere Kühlwirkung auszeichnen sollen.

Erfindungsgemäß können Zubereitungen, welche die erfindungsgemäßen Wirkstoffkombinationen enthalten, übliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Sofern die kosmetische oder dermatologische Zubereitung eine Lösung oder Lotion darstellt, können als Lösungsmittel verwendet werden: Wasser oder wäßrige Lösungen, ferner Öle, wie Triglyceride der Caprin- oder der Caprylsäure, vorzugsweise aber Rizinusöl, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, aber auch Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet, so wie bei alkoholischen Lösungsmitteln Wasser ein vorteilhafter weiterer Bestandteil sein kann.

Kosmetische und dermatologische Zubereitungen gemäß der Erfindung, auch z.B. zum Schutze der Haut vor UV-Strahlen, können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ ÖI-in-Wasser (O/W), oder eine multiple Emulsionen, beispielsweise vom Typ Wasserin-ÖI-in-Wasser (W/O/W), ÖI-in-Wasser-in-ÖI (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Die erfindungsgemäßen Wirkstoffe können auch besonders vorteilhaft in Mikroemulsionen, beispielsweise wie in der deutschen Offenlegungsschrift DE-195 9 079 beschrieben, verwendet werden.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Die folgenden Beispiele sollen die Verkörperungen der vorliegenden Erfindungen verdeutlichen.

| **Beispiel 1** O/W-selbstbräunungscreme für die Nacht | **Gel.-%** |
|---|---|
| Polyglyceryl-3-Methylglucosedistearat | 2 |
| Myristylmyristat | 2 |
| Stearylalkohol | 4 |
| Caprylsäure/Caprinsäuretriglyceride | 3 |
| Ethylhexylkokosfettsäureester | 2 |
| Glycerylstearat | 2 |
| Tocopherylacetat | 1 |
| Natriumascorbylphosphat | 0,1 |
| Glycyrrhetinsäure | 0,3 |
| p-Hydroxybenzoesäurealkylester (Paraben) | 0,6 |
| Carbomer | 0,2 |
| Carrageenan | 0,2 |
| EDTA | 0,2 |
| Butylenglycol | |
| Glycerin | 10 |
| wasser- und/oder öllösliche Farbstoffe | 0,05 |
| Füllstoffe/ Additive (SIO₂, BHT) | 0,2 |
| Parfüm | q.s. |
| Wasser | ad 100 |

| **Beispiel 2** O/W-Selbstbräunungs-Tagescreme | **Gew.-%** |
|---|---|
| Polyglyceryl-3-Methylglucosedistearat | 3 |
| Cetearylalkohol | 3 |
| Sheabutter | 1 |
| C₁₂₋₁₅ Alkylbenzoat | 3 |
| Caprylsäure/Caprinsäuretriglyceride | 2 |
| Paraffinöl | 2 |
| Macadamiaöl | 1 |
| Dicaprylylcarbonat | 2 |
| Ethylhexylmethoxycinnamat | 3 |
| Ethylhexyltriazon | 1 |
| Butylmethoxydibenzoylmethan | 2 |
| Glycyrrhetinsäure | 0.5 |
| Citronensäure, Natriumsalz | 0.1 |
| Phenoxyethanol | 0,5 |
| p-Hydroxybenzoesäurealkylester (Paraben) | 0,3 |
| Hexamidindiisethionat | 0,04 |
| 1,3-Dimethytol-5,5-dimethyl-hydantoin(DMDM Hydantoin) | 0,1 |
| EDTA | 0,2 |
| Carbomer | 0,3 |
| Glycerin | 8 |
| Additive (SiO₂, BHT) | 0,2 |
| Parfüm | q.s. |
| Wasser | ad 100 |

| **Beispiel 3** Sonnenschutzcreme mit Bräunungseffekt | **Gew.-%** |
|---|---|
| Sorbitanstearat | 3 |
| Polyglyceryl-3-Methylglucosedistearat | 1 |
| Cetearylalkohol | 2 |
| C₁₂₋₁₅ Alkylbenzoat | 3 |
| Cocosglyceride | 2 |
| Octyldodecanol | 3 |
| Hydrierte Kokosfettglyceride (Hydrogenated Coco Glycerides) | 3 |
| Ethylhexylmethoxycinnamat | 7 |
| Phenylbenzimidazol sulfonsäure | 2 |
| Butylmethoxydibenzoylmethan | 2 |
| Natriumascorbylphosphat | 0,1 |
| Tocophol | 0,1 |
| Glycyrrhetinsäure | 0,2 |
| Methylpropandiol | 2 |
| Phenoxyethanol | 0,5 |
| p-Hydroxybenzoesäurealkylester (Paraben) | 0,2 |
| Diazolidinylharnstoff | 0,1 |
| Tapiocastärke | 2 |
| Acrylacrylatcrosspolymer | 0,3 |
| Natriumacrylat | 0,1 |
| Glycerin | 7 |
| Additive (BHT, Iminodisuccinat) | 0,3 |
| Parfüm | q.s. |
| Wasser | ad 100 |

| **Beispiel 4** After Shave Gel | **Gew.-%** |
|---|---|
| Polyglyceryl-3-Methylglucosedistearat | 1,0 |
| Cyclometicone | 2,0 |
| Octyldodecanol | 1,0 |
| Dicaprylylcarbonat | 3,0 |
| Glycerylstearat | 1,0 |
| Glycyrrhetinsäure | 0,2 |
| Ethanol | 5,0 |
| Phenoxyethanol | 0,5 |
| p-Hydroxybenzoesäurealkylester (Paraben) | 0,4 |
| Carbomer | 0,3 |
| Distärkephosphat | 1,0 |
| Glycerin | 3,0 |
| Parfüm | q.s. |
| Wasser | ad 100 |

| **Beispiel 5** O/W Creme | **Gew.-%** |
|---|---|
| Polyglyceryl-3-Methylglucosedistearat | 3 |
| Stearylalkohol | 3 |
| C₁₂₋₁₅ Alkylbenzoat | 3 |
| Butylenglycol Dicaprylat/Dicaprat | 2 |
| Caprylsäure/Caprinsäuretriglyceride | 3 |
| Hydriertes Polydecen | 2 |
| Dimethylpolysiloxan (Dimethicon) | 1 |
| Vaseline | 1 |
| Bis-Ethylhexyloxyphenolmethoxyphenyltriazin | 1 |
| Ethylhexylmethoxycinnamat | 5 |
| Glycyrrhetinsäure | 0,5 |
| Natriumascorbylphosphat | 0,1 |
| Glucosylrutin | |
| Phenoxyethanol | 0,4 |
| Butylenglycol | 3 |
| p-Hydroxybenzoesäurealkylester (Paraben) | 0,4 |
| Carrageenan | 0,1 |
| Carbomer | 0,2 |
| Glycerin | 5 |
| Additive (Talkum, BHT) | 0,2 |
| Parfüm | q.s. |
| Wasser | ad 100 |

## Patentansprüche

1. Kosmetische Zubereitungen, umfassend (i) Glycyrrhizin und/oder Glycyrrhetinsäure und (ii) Polyglyceryl (3)-Methylglucosedisdistearat.

2. Zubereitungen nach Anspruch 1, **dadurch gekennzeichnet, dass** sie 0,0001 bis 0,5 Gew.-%, insbesondere 0,001 bis 0,5 Gew.-%, ganz besonders bevorzugt 0,005 bis 0,5 Gew.-% Glycyrrhizin und/oder Glycyrrhetinsäure enthalten, bezogen auf das Gesamtgewicht der Zubereitungen.

3. Verwendung von Wirkstoffkombinationen, umfassend
(i) Glycyrrhizin und/oder Glycyrrhetinsäure und
(ii) Polyglyceryl (3)-Methylglucosedistearat
zur Herstellung von Zubereitungen zur Verstärkung der Pigmentierung der humanen Haut

4. Verwendung nach Anspruch 3, wobei Glycyrrhizin und/oder Glycyrrhetinsäure in Konzentration von 0,0001 bis 0,5 Gew.-%, insbesondere 0,001 bis 0,5 Gew.-%, ganz besonders bevorzugt 0,005 bis 0,5 Gew.-% vorliegt oder vorliegen, bezogen auf das Gesamtgewicht der Zubereitungen.

## Claims

1. Cosmetic preparations comprising (i) glycyrrhizin and/or glycyrrhetinic acid and (ii) polyglyceryl (3) methylglucose distearate.

2. Preparations according to Claim 1, **characterized in that** they comprise 0.0001 to 0.5% by weight, in particular 0.001 to 0.5% by weight, very particularly preferably 0.005 to 0.5% by weight, of glycyrrhizin and/or glycyrrhetinic acid, based on the total weight of the preparations.

3. Use of active ingredient combinations comprising
(i) glycyrrhizin and/or glycyrrhetinic acid and
(ii) polyglyceryl (3) methylglucose distearate
for producing preparations for increasing the pigmentation in human skin.

4. Use according to Claim 3, where glycyrrhizin and/or glycyrrhetinic acid is or are present in concentrations of from 0.0001 to 0.5% by weight, in particular 0.001 to 0.5% by weight, very particularly preferably 0.005 to 0.5% by weight, based on the total weight of the preparations.

## Revendications

1. Préparations cosmétiques, comprenant (i) de la glycyrrhizine et/ou de l'acide glycyrrhétinique et (ii) du (3)-méthylgucosedistéarate de polyglycéryle.

2. Préparations selon la revendication 1,
**caractérisées en ce qu'**elles contiennent de 0,0001 à 0,5 % en poids, en particulier de 0,001 à 0,5 % en poids, de façon particulièrement préférée de 0,005 à 0,5 % en poids de glycyrrhizine et/ou d'acide glycyrrhétinique, par rapport au poids total des préparations.

3. Utilisation d'associations de substances actives, comprenant
(i) de la glycyrrhizine et/ou de l'acide glycyrrhétinique et
(ii) du (3)-méthylgucosedistéarate de polyglycéryle
pour la fabrication de préparations destinées à accentuer la pigmentation de la peau humaine.

4. Utilisation selon la revendication 3, dans laquelle est ou sont présent(s) de la glycyrrhizine et/ou de l'acide glycyrrhétinique à des concentrations de 0,0001 à 0,5 % en poids, en particulier de 0,001 à 0,5 % en poids, de façon particulièrement préférée de 0,005 à 0,5 % en poids, par rapport au poids total des préparations.
